# EUROPEAN PATENT APPLICATION

(11) **EP 4 736 899 A1**
(43) Date of publication of application: **06.05.2026**
(21) Application number: 25212397.1
(22) Date of filing: 30.10.2025
(51) Int. Cl.: A61L 31/04, A61L 31/10, A61L 31/16, C08L 5/08

(54) **INCISE DRAPE AND MANUFACTURING METHOD THEREOF**

(30) Priority: 04.11.2024 KR 20240154587; 23.06.2025 KR 20250082432
(71) Applicant: Endovision Co., Ltd., Daegu 41061 (KR)
(72) Inventor: JUNG, Min Ho, 42758 Daegu (KR)
(74) Representative: Viering, Jentschura & Partner mbB Patent- und Rechtsanwälte

(57) **Abstract**

Proposed is an incise drape including a base layer, and an adhesive layer formed on the base layer and containing acid-treated chitosan, povidone-iodine, and acrylate copolymer, wherein the adhesive layer contains less than 1 part by weight of povidone-iodine, and acid-treated chitosan in an amount of 1.5 to 5 parts by weight relative to povidone-iodine.

## Description

### CROSS REFERENCE TO RELATED APPLICATION

The present application claims priority to Korean Patent Application No. 10-2024-0154587, filed November 04, 2024 and Korean Patent Application No. 10-2025-0082432, filed June 23, 2025, the entire contents of which are incorporated herein for all purposes by this reference.

### BACKGROUND

### Technical Field

The present disclosure relates to a surgical incise drape with improved antimicrobial activity, and a manufacturing method thereof.

### Description of the Related Art

Incise drapes are used during surgery to protect the incision area from infection and other factors. Typically, an incise drape is composed of a base layer and an adhesive surface formed on one side of the base layer. An incise drape configured as above has an adhesive surface to be adhered to the skin and be cut through along with the skin by means of a surgical instrument.

The primary purpose of surgical incise drapes is to create a sterile barrier that prevents germs and bacteria from reaching the incision site. Because the skin is typically disinfected rather than sterilized during surgical preparation, bacteria can still remain on the skin and be transferred to the incision site during the incision. Incise drapes can be used to reduce infection at the surgical site by trapping skin microorganisms and providing a lasting antimicrobial effect. Furthermore, by stabilizing the skin and securing the incision site, the incise drapes can prevent an incision from opening or damage to the underlying tissue.

Surgical incise drapes use povidone-iodine (PVP-I) or chlorhexidine to provide antimicrobial properties.

Chlorhexidine has high antibacterial and antifungal activity with low skin irritation, making it a common alternative or supplement to povidone-iodine. However, chlorhexidine has limitations against some viruses and fungi on its own.

Povidone-iodine is a broad-spectrum antimicrobial that is effective against germs, bacteria, viruses, and fungi. Povidone-iodine's strong sterilizing power and rapid action speed allow it to exert a powerful sterilizing effect within seconds to minutes. Povidone-iodine is a versatile antiseptic used for disinfection, wound care, and medical instrument sterilization.

Povidone-iodine has different antimicrobial effects and clinical uses depending on its concentration. Povidone-iodine at lower concentrations has a milder antimicrobial effect, but it causes less skin irritation and leaves no residue on the skin after use, and does not significantly interfere with wound healing. Because of these properties, it is widely used in wound treatment patches, wound dressings, and skin disinfectants. High concentrations of povidone-iodine exhibit strong antimicrobial effects, making it used for surgical drapes and medical device disinfection to prevent surgical site infections. However, the high concentration can cause skin irritation, and prolonged use may lead to adverse effects such as burns and cytotoxicity.

For surgical incise drapes, high concentrations of povidone-iodine need to be used to enhance the antimicrobial effect since the incise drapes are used by directly adhering to the skin. However, using high concentrations of povidone-iodine for long periods can cause skin irritation like dryness, redness, or stinging, as described above, and its powerful antimicrobial action can be toxic to both bacteria and normal cells, potentially delaying wound healing if it penetrates open wounds or incisions. Moreover, some patients have been reported to develop allergic reactions to iodine.

To address these issues, various formulation strategies have been explored to maintain a low concentration of povidone-iodine while still achieving high antimicrobial efficacy by combining povidone-iodine with other antimicrobial agents. However, in most of these formulations, the concentration of povidone-iodine has not been reduced to below 1%, which is the threshold typically associated with minimal skin irritation. Thus, incise drapes still contain high concentrations of povidone-iodine, which carries the risk of skin irritation and inflammation.

Accordingly, there is a need to develop an incise drape that can have high antimicrobial activity even with low concentrations of povidone-iodine while minimizing skin irritation.

### SUMMARY

The present disclosure aims to provide an incise drape that has high antimicrobial activity even with low concentrations of povidone-iodine by containing acid-treated chitosan, and a method of manufacturing the incise drape.

In order to achieve the above objective, according to an aspect of the present disclosure, there is provided an incise drape including: a base layer; and an adhesive layer formed on the base layer and configured to contain acid-treated chitosan, povidone-iodine, and acrylate copolymer, wherein the adhesive layer may contain less than 1 part by weight of povidone-iodine based on the total weight of the adhesive layer, and acid-treated chitosan in an amount of 1.5 to 5 parts by weight per 1 part by weight of povidone-iodine.

In an embodiment of the present disclosure, the chitosan may include mushroom-derived chitosan.

In an embodiment of the present disclosure, the mushroom-derived chitosan may have a molecular weight between 3,000 to 30,000 Da.

In an embodiment of the present disclosure, the acrylate copolymer may include at least one selected from the group consisting of vinyl acrylate copolymer, methacrylate copolymer, stearyl acrylate copolymer, butyl acrylate copolymer, alkyl acrylate copolymer, acrylic acid and acrylate copolymer, acrylate-polyester copolymer, acrylate-polyurethane copolymer, and acrylate-silicone copolymer.

In an embodiment of the present disclosure, the base layer may include a polyurethane layer formed atop a polyolefin film.

In an embodiment of the present disclosure, the drape may include a release liner film on one or both sides thereof.

In order to achieve the above objective, according to an aspect of the present disclosure, there is provided a manufacturing method of an incise drape, the method including: S1) preparing a base layer; S2) manufacturing an adhesive layer by preparing a mixed solution containing acid-treated chitosan, povidone-iodine, and acrylate copolymer and applying the mixture to the base layer; and S3) drying, wherein the mixed solution may contain acid-treated chitosan, povidone-iodine, and acrylate copolymer in a weight ratio of 0.1 to 5: 0.1 to 10: 75 to 99.

In an embodiment of the present disclosure, the step S1) may include forming the base layer by applying 40 to 60 mL of polyurethane per 1 m² of polyolefin film.

In an embodiment of the present disclosure, the step S2) may include: S2-1) preparing the mixed solution by combining the acid-treated chitosan, povidone-iodine, and acrylate copolymer and stirring the mixture at 30 to 40 °C for 80 to 100 minutes; S2-2) heating the mixed solution to 70 to 90 °C and mixing for an additional 50 to 90 minutes; and S2-3) forming the adhesive layer by stirring the mixed solution for 1 to 10 hours by reducing temperature to 30 to 40 °C and then applying 80 to 110 mL of the stirred mixed solution per 1 m² of the base layer.

In an embodiment of the present disclosure, the acid-treated chitosan may be prepared by immersing mushroom-derived chitosan in an acid solution to form an acid-treated chitosan mixture, followed by washing with at least one of an alcohol or a surfactant, and then drying the resulting material.

In an embodiment of the present disclosure, the acrylate copolymer may include at least one selected from the group consisting of polyacrylic acid, acrylic acid-alkyl acrylate copolymer, and polyacrylamide.

According to the present disclosure, an incise drape with high antimicrobial activity even with low concentrations of povidone-iodine by containing acid-treated chitosan, and a method of manufacturing the incise drape can be provided.

The incise drape of the present disclosure can minimize irritation to the skin even when used for long-term surgery, and can thus be easily applied to patients with skin inflammation or trauma.

The incise drape of the present disclosure can exhibit higher chemical resistance and durability than other surgical drapes because a base layer includes polyurethane formed on polyethylene.

### BRIEF DESCRIPTION OF THE DRAWINGS

FIG. 1 shows an incise drape according to an embodiment of the present disclosure.
FIG. 2 shows an incise drape according to another embodiment of the present disclosure.
FIG. 3 shows an incise drape according to still another embodiment of the present disclosure.

### DETAILED DESCRIPTION

Povidone-iodine (PVP-I) is a broad-spectrum antimicrobial agent with antibacterial, antiviral, and antifungal properties. Its efficacy, combined with a well-established safety profile, has contributed to its widespread use for antimicrobial and disinfection purposes. Povidone-iodine exhibits different properties depending on its concentration. At low concentrations (less than 1%), povidone-iodine is non-irritating, does not leave a residue on the skin after direct contact, and does not interfere with wound healing. These characteristics make it suitable for use as a wound dressing, topical antiseptic, or disinfectant. On the other hand, high concentrations (3 to 12%) of povidone-iodine offer stronger disinfection and antimicrobial effects than low concentrations, but carry significant risks like skin irritation, contact dermatitis, and potential damage to normal cells in trauma or incision areas, which can slow healing.

Conventional surgical incise drapes are manufactured to contain 3 to 12% povidone-iodine concentrations, as this higher concentration ensures a potent antimicrobial effect to prevent the penetration of bacteria or germs during surgical incisions, despite the drapes being applied directly to the skin. Because high concentrations of povidone-iodine continuously irritate the skin during the surgery, people with sensitive skin, chronic skin diseases, or a history of iodine allergies are likely to experience skin irritation and mild inflammatory reactions.

Accordingly, the inventors of the present disclosure developed an incise drape that satisfies the antimicrobial activity required for surgical operations while maintaining the concentration of povidone-iodine at a level that minimizes skin irritation by using chitosan and povidone-iodine together in a surgical incise drape, thereby completing the present disclosure.

The present disclosure relates to an incise drape including: a base layer; and an adhesive layer formed on the base layer and containing acid-treated chitosan, povidone-iodine, and acrylate copolymer. The adhesive layer contains less than 1 part by weight of povidone-iodine, and acid-treated chitosan in an amount of 1.5 to 5 parts by weight relative to povidone-iodine.

The incise drape according to an embodiment of the present disclosure has a remarkable effect of satisfying the antimicrobial activity required for a surgical incise drape, even though the drape contains a lower concentration of povidone-iodine to avoid skin irritation, by including acid-treated chitosan and povidone-iodine together as described above. This effect is achieved by using acid-treated chitosan, which exhibits enhanced antimicrobial activity than general chitosan, together with povidone-iodine.

First, the adhesive layer is a layer that adheres to the skin and exhibits antimicrobial activity. As described above, the adhesive layer includes acid-treated chitosan, povidone-iodine, and an acrylate copolymer. The povidone-iodine may be included in an amount of less than 1 part by weight based on the total adhesive layer weight, and the acid-treated chitosan may be included in an amount of 1 to 10 parts by weight, specifically 1.5 to 5 parts by weight, based on the povidone-iodine.

Acid-treated chitosan is chitosan that has been dissolved in an acidic solution, which protonates its amino groups, converting it into a cationic (positively charged) form. Chitosan in its native molecular form has low solubility in most solvents, which limits its ability to exhibit defective antimicrobial activity. On the other hand, acid-treated chitosan contains protonated amine groups (-NH₃⁺) within its molecular structure, which impart a positive charge. This increases solubility and enhances electrostatic interactions with negatively charged bacteria cell membranes, thereby potentially improving its antimicrobial activity.

Chitosan may include chitosan derived from Aspergillus niger (black aspergillus) or mushrooms, and may have a molecular weight ranging from 3,000 to 30,000 Da, specifically 5,000 to 25,000 Da.

Traditionally, chitosan used as an antibacterial agent in incise drapes was derived primarily from crustaceans like crabs and shrimp. However, crustacean-derived chitosan has a high molecular weight, making it difficult to dissolve in many solvents and to penetrate bacterial cell membranes, resulting in lower antimicrobial activity compared to low-molecular-weight chitosan. Moreover, it was difficult to use for patients with shellfish allergies or vegans. In contrast, chitosan derived from Aspergillus niger (black aspergillus) or mushrooms has a lower molecular weight (3,000 to 30,000, specifically 5,000 to 25,000) than that from crustaceans. This allows it to dissolve more easily and penetrate bacterial cell membranes more effectively, resulting in superior antimicrobial activity compared to crustacean-derived chitosan.

That is, since the adhesive layer according to an embodiment of the present disclosure uses chitosan with maximized antimicrobial activity by acid-treating low molecular weight chitosan together with povidone-iodine, the adhesive layer can exhibit high antimicrobial activity despite containing povidone-iodine at a relatively low concentration that does not irritate the skin.

Povidone-iodine and acid-treated chitosan included in the adhesive layer may contain povidone-iodine in an amount of less than 1 part by weight based on the total weight of the adhesive layer, and acid-treated chitosan in an amount of 1.5 to 5 parts by weight based on 1 part by weight of povidone-iodine, in order to have high antimicrobial activity while minimizing skin irritation.

As described above, povidone-iodine should be included in an amount of less than 1 part by weight relative to the total adhesive layer weight to minimize skin irritation, and in order to have high antimicrobial activity, it is advantageous to include acid-treated chitosan in an amount of 1.5 to 10 parts by weight, specifically 1.5 to 5 parts by weight, relative to povidone-iodine.

Since acid-treated chitosan can modulate the bactericidal action of povidone-iodine, povidone-iodine and acid-treated chitosan should be included within the above-described ranges so that the bactericidal action of povidone-iodine and the antibacterial action of chitosan can work in balance to achieve the intended effects of the present disclosure. That is, as povidone-iodine and acid-treated chitosan are included in the adhesive layer in the above-described range, chitosan's antibacterial action can be maximized without inhibiting the disinfecting action of povidone-iodine.

The acrylate copolymer may include at least one selected from the group consisting of vinyl acrylate copolymer, methacrylate copolymer, stearyl acrylate copolymer, butyl acrylate copolymer, alkyl acrylate copolymer, acrylic acid and acrylate copolymer, acrylate-polyester copolymer, acrylate-polyurethane copolymer, and acrylate-silicone copolymer.

The acrylate copolymer functions as a binder to fix chitosan and povidone-iodine in place and provides adhesive properties that enables secure attachment to the skin.

The acrylate copolymer may be included in an amount of 90 to 99 parts by weight, specifically 95 to 99 parts by weight, based on the total weight of the adhesive layer.

The base layer may be a single polyolefin film that is conventionally used in surgical drapes, and may include polyurethane formed on a polyolefin film. If the base layer additionally includes a polyurethane layer, the base layer can better block skin fluids or blood, thereby further reducing the risk of infection caused by germs or bacteria. The polyurethane layer within the base layer also helps to regulate an appropriate water vapor transmission rate (WVTR), maintaining skin humidity to prevent skin irritation while enhancing the film's adhesion and flexibility, leading to a more stable attachment to the skin.

According to an embodiment, the base layer including polyurethane formed on the polyolefin film may have a WVTR of 1,500 to 2,500 g/m²/24h, specifically 1,900 to 2,100 g/m²/24h, as measured according to ASTM F 1249.

The incise drape described above may be stored with a release liner film attached to one or both sides thereof before being applied to the surgical site.

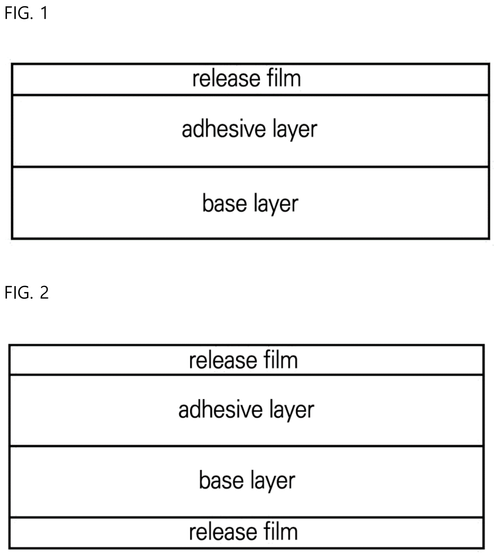

Referring to FIGS. 1 and 2, the above-mentioned release film may be formed on only one side of the adhesive layer of the incise drape, or may be formed on both sides of the base layer and the adhesive layer. When the release film is provided only on one side of the adhesive layer of the incise drape, the release film can be used by removing the release film on the adhesive layer before surgery and then attaching the release film to the surgical site. When the release film is provided on both the adhesive layer and the base layer, the release film can be used by removing the release film on the adhesive layer, attaching the adhesive layer to the surgical site, and removing the release film on the base layer.

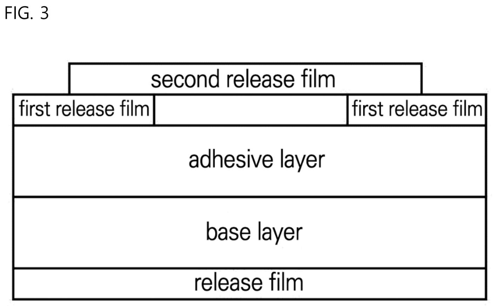

In addition, referring to FIG. 3, the release film on the adhesive layer may be manufactured to include: a first release film formed at each end of the adhesive layer by being spaced apart from each other as shown in FIG. 3; and a second release film formed on the spaced apart first release films to connect the first release films. This helps to attach the incise drape more closely to the skin. After removing the second release film and attaching the exposed adhesive layer portion to the skin, the first release films are removed and the remaining adhesive layer portion can be attached to the skin.

The present disclosure provides a manufacturing method of an incise drape, the method including: preparing S1 a base layer; manufacturing S2 an adhesive layer by preparing a mixed solution containing acid-treated chitosan, povidone-iodine, and acrylate copolymer and applying the mixture to the base layer; and drying S3. The mixed solution contains acid-treated chitosan, povidone-iodine, and acrylate copolymer in a weight ratio of 0.1 to 5: 0.1 to 10: 75 to 99.

According to the manufacturing method of an incise drape of the present disclosure, an incise drape with excellent antimicrobial activity even with low concentrations of povidone-iodine by containing acid-treated chitosan may be manufactured.

S1 is a step of preparing a base layer. The base layer may be manufactured and used or purchased from a commercially available source. The base layer is not limited to a polyolefin film, and the thickness thereof may be 0.01 to 0.08 mm, specifically 0.02 to 0.04 mm.

In an embodiment, step S1 may include manufacturing a base layer by forming a polyurethane film such that 40 to 60 mL of polyurethane is applied per 1 m² of the polyolefin film. In this case, as the polyurethane is formed in the base layer in the above-described range, the base layer may exhibit excellent water vapor transmission rate and tensile strength.

The base layer including polyurethane formed on the polyolefin film may satisfy a water vapor transmission rate of 1,500 to 2,500, specifically 1,900 to 2,100 g/m²/24h, based on ASTM F 1249.

S2 is a step of manufacturing an adhesive layer by preparing a mixed solution containing acid-treated chitosan, povidone-iodine, and acrylate copolymer and applying the mixture to the base layer. At this time, acid-treated chitosan, povidone-iodine, and acrylate copolymer may be included in a weight ratio of 0.1 to 5: 0.1 to 10: 75 to 99.

As the above adhesive layer contains acid-treated chitosan, povidone-iodine and acrylate copolymer within the above-described range, the adhesive layer manufactured by drying later contains less than 1 part by weight of povidone-iodine based on the total adhesive layer weight, and contains 1.5 to 10 parts by weight, specifically 1.5 to 5 parts by weight, of acid-treated chitosan based on 1 part by weight of povidone-iodine. Thus, it is possible to manufacture the adhesive layer that exhibits excellent antimicrobial activity without causing irritation or inflammation to the skin.

Acid-treated chitosan may be prepared by immersing mushroom-derived chitosan in an acid solution to form an acid-treated chitosan mixture, followed by washing with at least one of an alcohol or a surfactant, and then drying the resulting material.

The acid solution may contain an acidic compound and an alcohol solvent. Specifically, the acidic compound and the alcohol solvent may be mixed in a weight ratio of 7:93 to 10:90.

The acidic compound may include, for example, one or more acidic substances selected from the group consisting of hydrochloric acid, acetic acid, ascorbic acid, nitric acid, sulfuric acid, hydrofluoric acid and phosphoric acid, and specifically, may be one or more selected from hydrochloric acid, acetic acid, or ascorbic acid.

The alcohol solvent is not limited as long as it can dissolve an acidic compound, but may be, for example, one or more selected from the group consisting of methanol, ethanol, propanol, and butanol, and specifically, may be ethanol.

The immersion may be performed at a temperature of 40 to 70 °C for 20 to 40 minutes. In case that the temperature is below 40 °C or the immersion time is less than 20 minutes, the introduction of cations to the amine groups of chitosan molecules is insufficient, thereby limiting the efficacy of the resulting material as a disinfectant. In case that the temperature exceeds 70 °C or the immersion time exceeds 40 minutes, the acid treatment may proceed excessively, potentially weakening the physical properties of the chitosan and consequently reducing its adsorption performance.

The washing may include a first washing step using alcohol, a second washing step using alcohol, and a third washing step using an alcohol and a surfactant at a weight ratio of 99:1 to 99.9:0.1.

Acidic compounds remaining during the acid treatment process may be removed through primary and secondary washing with alcohol. As the alcohol, the same type of alcohol used in the acid treatment step can be used, specifically ethanol.

Each of the first and second steps may be performed at 40 to 70 °C for a duration of 50 to 80 minutes. If the washing temperature is too low or the washing time is too short outside the above range, there is a risk that the washing will not be sufficient and the acidic components remaining in the chitosan may cause excessive irritation to the wound area. If the washing temperature is too high or the washing time is too long, the acid-treated chitosan may be physically and chemically damaged, which is not desirable.

The third washing step may improve wetting properties by modifying the surface of chitosan to be more hydrophilic through the use of a mixture of an alcohol and a surfactant in a weight ratio of 99:1 to 99.9:0.1. This treatment creates a continuous moist environment when applied to the wound site, enhancing absorption and forming a protective film over the sterilized area to minimize the risk of infection.

In case that the content of the surfactant is less than 0.1 by weight, sufficient surface modification cannot be achieved and a hydrophilic chitosan agent cannot be obtained, and in case that the content exceeds 1, the chitosan surface may be damaged, which is not preferable.

The surfactant is not limited as long as it is a cationic, anionic, or non-ionic surfactant, but the surfactant may be, for example, one or more selected from a Tween-type non-ionic surfactant and an LE-type non-ionic surfactant.

The first, second, third washing steps may be performed sequentially, and more specifically, by drying after each washing, continuous and unnecessary acid treatment due to remaining acidic compounds may be minimized, thereby minimizing damage to a product and maintaining proper performance.

The drying may be performed at 35 to 55 °C. Through the drying, residual components such as surfactants remaining in chitosan may be effectively removed.

The chitosan may include chitosan derived from Aspergillus niger (black aspergillus) or mushrooms. As described above, chitosan derived from black aspergillus or mushrooms possesses a lower molecular weight than conventional crustacean-derived chitosan, which may contribute to its higher antimicrobial efficacy.

The acrylate copolymer functions to bind povidone-iodine and acid-treated chitosan and further imparts adhesive properties to the adhesive layer. Since the types thereof have been described above, detailed descriptions are omitted.

In an embodiment, the step S2 may include: preparing S2-1 a mixed solution by combining acid-treated chitosan, povidone-iodine, and acrylate copolymer and stirring the mixture at 30 to 40 °C for 80 to 100 minutes; heating S2-2 the mixed solution to 70 to 90 °C and mixing for an additional 50 to 90 minutes; and forming S2-3 an adhesive layer by stirring the mixed solution for 1 to 10 hours by reducing the temperature to 30 to 40 °C and then applying 80 to 110 mL of the stirred mixed solution per 1 m² of the base layer.

S2-1 is a step of preparing a mixed solution by stirring acid-treated chitosan, povidone-iodine, and acrylate copolymer so that the acid-treated chitosan and povidone-iodine are sufficiently dispersed in the acrylate copolymer.

S2-2 is a step of crosslinking the acrylate copolymer by heating the mixed solution in which acid-treated chitosan, povidone-iodine, and acrylate copolymer are uniformly dispersed.

S2-3 is a step in which the crosslinking is completed, the crosslinked acrylate copolymer, the acid-treated chitosan, and povidone-iodine are mixed again, allowing the acid-treated chitosan and povidone-iodine to be uniformly dispersed within the crosslinked acrylate copolymer, and then the resulting mixture is applied to the base layer to form an adhesive layer. When applying the mixture to the base layer, 70 to 130 mL, specifically 80 to 110 mL, of the mixture per 1 m² of the base layer is applied to form an adhesive layer. As the adhesive layer is formed by applying the mixture within the above-described range, appropriate skin adhesion and antimicrobial activity may be achieved.

Since the step S2 includes the steps S2-1 to S2-3, the acid-treated chitosan and povidone-iodine are uniformly distributed in the adhesive layer, so that antimicrobial activity may be evenly exhibited throughout the adhesive layer.

S3 is a step of drying the adhesive layer manufactured in step S2. This step removes the solvent and impurities contained in the acrylate copolymer and fixes the acid-treated chitosan and povidone-iodine to the acrylate copolymer.

Hereinafter, the present disclosure will be described in more detail using the following Examples.

### (Example 1)

A base layer is manufactured by applying 50 mL of PU emulsion per 1 m² of light release PET, followed by coating with a coating machine to a thickness of 0.03 mm.

Mushroom-derived chitosan powder was mixed with 7% HCl and 93% EtOH and stirred on a hot plate at 50 °C for 30 minutes to acid-treat the chitosan. Afterwards, the acid-treated mushroom-derived chitosan was obtained by performing the first washing step (100% EtOH, hot plate temperature: 50 °C, time: 1 hr), the second washing step (100% EtOH, hot plate temperature: 50 °C, 1 hr), and the third washing step (0.125% Tween 20 + 99.875% EtOH, hot plate temperature: 50 °C, time: 1 hr) before drying (temperature: 46 °C).

The acrylate copolymer, LOCTITE DURO-TAK (Henkel), was prepared, and the acid-treated mushroom-derived chitosan, povidone-iodine, and acrylate copolymer were mixed in a weight ratio of 1.3:0.7:98, respectively.

The mixed solution was stirred at 35 °C for 90 minutes, then heated to 85 °C and stirred for an additional hour. The solution was then cooled to 35 °C and applied at 100 mL per 1 m² onto the base layer to form a drape layer, which was then dried to manufacture a surgical incise drape.

### (Example 2)

The same procedure as in Example 1 was followed, except that chitosan, povidone-iodine, and acrylate copolymer were mixed in a weight ratio of 1.3: 0.8: 97.9.

### (Example 3)

The same procedure as in Example 1 was followed, except that chitosan, povidone-iodine, and acrylate copolymer were mixed in a weight ratio of 1.3: 0.9: 97.8.

### (Example 4)

The same procedure as in Example 1 was followed, except that chitosan, povidone-iodine, and acrylate copolymer were mixed in a weight ratio of 2: 0.7: 97.3.

### (Example 5)

The same procedure as in Example 1 was followed, except that chitosan, povidone-iodine, and acrylate copolymer were mixed in a weight ratio of 3: 0.7: 96.3.

### (Comparative Example 1)

The same procedure as in Example 1 was followed, except that chitosan, povidone-iodine, and acrylate copolymer were mixed in a weight ratio of 0.5: 0.7: 98.8.

### (Comparative Example 2)

The same procedure as in Example 1 was followed, except that chitosan, povidone-iodine, and acrylate copolymer were mixed in a weight ratio of 1.3: 3: 95.7.

### (Comparative Example 3)

The same procedure as in Example 1 was followed, except that povidone-iodine and acrylate copolymer were mixed in a weight ratio of 1:99.

### (Comparative Example 4)

The same procedure as in Example 1 was followed, except that chitosan and acrylate copolymer were mixed in a weight ratio of 1:99.

### (Experimental Example 1) Antimicrobial Test

The antimicrobial performance of the compositions according to Examples 1 to 3 was evaluated and shown in Table 1 below. To be specific, the concentration of bacteria at the time of initial inoculation and the concentration of bacteria measured 5 minutes later were compared according to the guidelines for evaluating topical antiseptic (over-the-counter drug) efficacy for four types of pathogens (Staphylococcus aureus, Escherichia coli, Streptococcus pneumoniae, and Pseudomonas aeruginosa), and the results are shown in Table 1 below.

**[Table 1]**

| Test items | | Test results | | | |
|---|---|---|---|---|---|
| Guidelines for evaluating topical antiseptic efficacy | Reduction rate of viable bacterial count (CFU) after 5 minutes | Control | Example 1 | Example 3 | Example 5 |
| Staphylococcus aureus ATCC 6538 | Reduction rate of viable bacterial count (CFU) after 5 minutes | 7.6X10³ | <10 99.9 | <10 99.9 | <10 99.9 |
| Escherichia coli ATCC 8739 | Reduction rate of viable bacterial count (CFU) after 5 minutes | 6.5X10³ | <10 99.9 | <10 99.9 | <10 99.9 |
| Klebsiella pneumoniaeATCC 4352 | Reduction rate of viable bacterial count (CFU) after 5 minutes | 4.7X10³ | <10 99.9 | <10 99.9 | <10 99.9 |
| P s e u d o m o n a s aeruginosaATCC 10145 | Reduction rate of viable bacterial count (CFU) after 5 minutes | 3.8X10³ | <10 99.9 | <10 99.9 | <10 99.9 |

As a result of the test, it was confirmed that when the ratio of acid-treated chitosan and povidone-iodine was 1 to 5:1 while the ratio of povidone-iodine was less than 1% according to an embodiment of the present disclosure, the bacterial reduction rate after 5 minutes for 4 types of pathogens was 99.9%, satisfying the guidelines for evaluating topical antiseptic efficacy, and exhibiting excellent antimicrobial properties.

That is, as confirmed in Table 1 above, Examples 1, 3, and 5 exhibited high antimicrobial activity, similar to conventional incise drapes containing 1% or more of povidone-iodine, even though Examples 1, 3, and 5 contained less than 1% of povidone-iodine, by additionally including acid-treated chitosan.

### (Experimental Example 2) Assessment of Cytotoxicity According to Content of Chitosan and Povidone-Iodine

The results of confirming cytotoxicity for Experimental Examples 1 to 5 and Comparative Examples 1 to 4 according to the present disclosure are shown in Table 2 below.

**[Table 2]**

| | Chitosan content (g) | PVP-I content (g) | Cytotoxicity |
|---|---|---|---|
| Example 1 | 1.3 | 0.7 | Non-cytotoxic |
| Example 2 | 1.3 | 0.8 | Non-cytotoxic |
| Example 3 | 1.3 | 0.9 | Non-cytotoxic |
| Example 4 | 2 | 0.7 | Non-cytotoxic |
| Example 5 | 3 | 0.7 | Non-cytotoxic |
| Comparative Example 1 | 0.5 | 0.7 | Non-cytotoxic |
| Comparative Example 2 | 1.3 | 1.5 | Cytotoxic |
| Comparative Example 3 | 0 | 1 | Cytotoxic |
| Comparative Example 4 | 1 | 0 | Non-cytotoxic |

Referring to Table 2 above, since povidone-iodine is included in less than 1 part by weight for the entire adhesive layer, Examples 1 to 5 and Comparative Example 1 are free of cytotoxicity, and Comparative Example 4, which does not include povidone-iodine and only contains chitosan, is also free of cytotoxicity, confirming its suitability for use as an adhesive layer.

On the other hand, Comparative Examples 2 and 3 were found to be cytotoxic and therefore unsuitable for use as an adhesive layer, as they contained povidone-iodine in an amount of 1 part by weight or more relative to the total weight.

### (Experimental Example 3) Antimicrobial Activity Test

A single colony of the test strain was inoculated into a 200-mL flask containing 10 mL of nutrient broth and incubated at 37 °C with stirring for 12 hours. The resulting culture was then transferred to a fresh 200-mL flask containing 10 mL of nutrient broth at an initial optical density (O.D.) of 0.1 and subcultured for 7 hours. Each test strain culture prepared as described above was adjusted to contain approximately 2 X 10⁸ cells per plate. Then, 13mL of non-solidified nutrient agar (cooled to 60 °C) was mixed with the bacterial suspension and poured into a 1000-mm Petri dish. The mixture was allowed to solidify completely. Next, 100 µL each of Examples 1 to 5 and Comparative Examples 1 and 4, which showed no cytotoxicity in Experimental Example 2 above, were applied to the plate. The plate was then incubated at 37°C for 12 hours, and the results are presented in Table 3.

**[Table 3]**

| Example 1 | Example 2 | Example 3 | Example 4 | Example 5 | Comparative Example 1 | Comparative Example 4 |
|---|---|---|---|---|---|---|
| 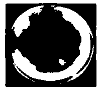 | 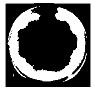 | 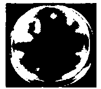 | 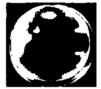 | 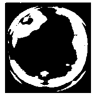 | 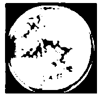 | 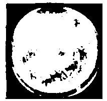 |

Referring to Table 3, it was confirmed that Examples 1 to 5 exhibited significantly greater antimicrobial properties compared to Comparative Examples 1 and 4. Referring to the results of Examples 1 to 3, when the content of chitosan was held constant, the antimicrobial activity increased with higher concentrations of povidone-iodine.

A comparison between Example 1 and Example 4 confirms that increasing the chitosan content, while maintaining a constant level of povidone-iodine, results in enhanced antimicrobial activity in Example 4.

A comparison between Example 4 and Example 5 confirms that when the povidone-iodine content was kept constant, the Petri dish of Example 5 was slightly more transparent than that of Example 4 due to the increased chitosan content; however, both exhibited a similar level of antimicrobial activity.

That is, the excellent antimicrobial properties of Examples 1 to 5, as shown in Table 3, were confirmed. As described above, when chitosan and povidone-iodine were combined in the specific ratios of the present disclosure, chitosan demonstrated a remarkable ability to maximize antimicrobial activity without inhibiting the disinfecting effect of povidone-iodine.

It was confirmed that antimicrobial properties of Comparative Examples 1 and 4 were poor because chitosan was mixed at too low a ratio relative to povidone-iodine in Comparative Example 1, and povidone-iodine was absent in Comparative Example 4.

### (Experimental Example 4) Skin Irritation Test Results

Thirty adults aged 20 to 50 years were selected as subjects. Drapes measuring 10 X 10 cm² from Examples 1 to 5 and Comparative Examples 1 to 4 were applied to the skin for 24 hours. Skin irritation was then assessed, and the average results are presented in Table 2 below. The skin irritation assessment score was assigned as follows: 0 points for no irritation (-), 0.5 points for minimal irritation (±), 1 point for mild irritation (+), 1.5 points for moderate irritation (++), and 2 points for severe irritation (+++). The average scores are presented in Table 4 below.

**[Table 4]**

| | Irritation score |
|---|---|
| Example 1 | 0 |
| Example 2 | 0 |
| Example 3 | 0 |
| Example 4 | 0 |
| Example 5 | 0 |
| Comparative Example 1 | 0.3 |
| Comparative Example 2 | 1.2 |
| Comparative Example 3 | 1.8 |
| Comparative Example 4 | 0 |

In the irritation evaluation, Examples 1 to 5 showed no irritation even after 24 hours of application. However, Comparative Example 1 exhibited a minimal irritation reaction, likely due to the higher povidone-iodine content relative to chitosan, with an average score of 0.3, and Comparative Examples 2 and 3 exhibited moderate to severe skin irritation, including redness, dryness, and stinging, due to the inclusion of povidone-iodine in amounts exceeding 1 part by weight relative to the total weight of the adhesive layer. In contrast, Comparative Example 4, which contained only chitosan, did not induce any skin irritation.

That is, Examples 1 to 5 of the present disclosure contained povidone-iodine and acid-treated chitosan in a weight ratio of 1:1.5 to 5. As a result, skin irritation was significantly reduced while maintaining comparable antimicrobial activity despite the lower concentration of povidone-iodine compared to Comparative Example 3 which contained povidone-iodine but no chitosan. This can be seen as a remarkable characteristic suitable for surgical incise drapes that need to be attached to the skin for long periods of time.

In addition, Comparative Example 2 contained an excessive amount of povidone-iodine, yet it caused less skin irritation than Comparative Example 3, which did not contain chitosan. This reduction in irritation is attributed to the inclusion of chitosan. However, as the povidone-iodine content increased, some skin irritation, such as redness and dryness, was still observed. Comparative Example 3, which did not contain chitosan, exhibited significant skin irritation due to the presence of povidone-iodine. Comparative Example 4, which contained only chitosan, caused no skin irritation. However, as shown in Experimental Example 3, its antimicrobial activity was significantly reduced.

However, in cases where the drape was applied to areas with pre-existing skin inflammation or trauma, severe irritation was observed in some instances, even with the drape corresponding to Comparative Example 1. That is, when applied to areas affected by inflammation or trauma, Comparative Example 1 could also cause notable skin irritation.

Therefore, it was confirmed that the drapes corresponding to Examples 1 to 5 provide excellent stability and antimicrobial properties, making them suitable for use as surgical incise drapes where concerns about incision site penetration and varying patient skin conditions should be taken into account.

Although the present disclosure has been described through specific matters and limited embodiments as described above, these are provided only to help a more general understanding of the present disclosure, and the present disclosure is not limited to the above embodiments, and those skilled in the art to which the present disclosure pertains can make various changes and modifications based on this description.

## Claims

1. An incise drape comprising:
a base layer; and
an adhesive layer formed on the base layer and configured to contain acid-treated chitosan, povidone-iodine, and acrylate copolymer,
wherein the adhesive layer contains the povidone-iodine in an amount of less than 0.7 to 1 part by weight based on a total weight of the adhesive layer, and the acid-treated chitosan in an amount of 1.5 to 5 parts by weight relative to the povidone-iodine.

2. The drape of claim 1, wherein the chitosan includes mushroom-derived chitosan.

3. The drape of claim 2, wherein the mushroom-derived chitosan has a molecular weight between 3,000 to 30,000 Da.

4. The drape of claim 1, wherein the acrylate copolymer comprises at least one selected from the group consisting of vinyl acrylate copolymer, methacrylate copolymer, stearyl acrylate copolymer, butyl acrylate copolymer, alkyl acrylate copolymer, acrylic acid and acrylate copolymer, acrylate-polyester copolymer, acrylate-polyurethane copolymer, and acrylate-silicone copolymer.

5. The drape of claim 1, wherein the base layer comprises a polyurethane layer formed atop a polyolefin film.

6. The drape of claim 1 comprises a release liner film on one or both sides thereof.

7. A manufacturing method of an incise drape, the method comprising:
S1) preparing a base layer;
S2) manufacturing an adhesive layer by preparing a mixed solution containing acid-treated chitosan, povidone-iodine, and acrylate copolymer and applying the mixture to the base layer; and
S3) drying,
wherein the mixed solution contains acid-treated chitosan, povidone-iodine, and acrylate copolymer in a weight ratio of 0.1 to 5: 0.1 to 10: 75 to 99.

8. The method of claim 7, wherein the step S1) comprises:
forming the base layer by applying 40 to 60 mL of polyurethane per 1 m² of polyolefin film.

9. The method of claim 7, wherein the step S2) comprises:
S2-1) preparing the mixed solution by combining the acid-treated chitosan, povidone-iodine, and acrylate copolymer and stirring the mixture at 30 to 40 °C for 80 to 100 minutes;
S2-2) heating the mixed solution to 70 to 90 °C and mixing for an additional 50 to 90 minutes; and
S2-3) forming the adhesive layer by stirring the mixed solution for 1 to 10 hours by reducing temperature to 30 to 40 °C and then applying 80 to 110 mL of the stirred mixed solution per 1 m² of the base layer.

10. The method of claim 7, wherein the acid-treated chitosan is prepared by immersing mushroom-derived chitosan in an acid solution to form an acid-treated chitosan mixture, followed by washing with at least one of an alcohol or a surfactant, and then drying the resulting material.

11. The method of claim 7, wherein the acrylate copolymer comprises at least one selected from the group consisting of vinyl acrylate copolymer, methacrylate copolymer, stearyl acrylate copolymer, butyl acrylate copolymer, alkyl acrylate copolymer, acrylic acid and acrylate copolymer, acrylate-polyester copolymer, acrylate-polyurethane copolymer, and acrylate-silicone copolymer.
